(19) Europäisches Patentamt
European Patent Office
Office européen des brevets

(11) EP 0 700 317 B1

(12) **EUROPEAN PATENT SPECIFICATION**

(45) Date of publication and mention of the grant of the patent:
**18.08.1999 Bulletin 1999/33**

(21) Application number: **95904553.5**

(22) Date of filing: **29.12.1994**

(51) Int. Cl.[6]: **B01J 23/36**, B01J 23/28, C07C 6/04, C07C 6/06

(86) International application number:
**PCT/FI94/00585**

(87) International publication number:
**WO 95/17956 (06.07.1995 Gazette 1995/29)**

# (54) CATALYST FOR METATHESIS REACTIONS AND USE OF IT

KATALYSATOR FÜR METATHESIS-REAKTIONEN UND DESSEN VERWENDUNG

CATALYSEUR DESTINE A DES REACTIONS DE METATHESE ET SON UTILISATION

(84) Designated Contracting States:
**AT BE CH DE FR GB IT LI NL SE**

(30) Priority: **30.12.1993 FI 935941**

(43) Date of publication of application:
**13.03.1996 Bulletin 1996/11**

(73) Proprietor: **NESTE OY**
**02150 Espoo (FI)**

(72) Inventors:
- **LINNA, Anja**
  **FIN-06150 Porvoo (FI)**
- **HIETALA, Jukka**
  **FIN-06400 Porvoo (FI)**
- **KNUUTTILA, Pekka**
  **FIN-06400 Porvoo (FI)**

(74) Representative:
**Bühling, Gerhard, Dipl.-Chem. et al**
**Patentanwaltsbüro**
**Tiedtke-Bühling-Kinne & Partner**
**Bavariaring 4**
**80336 München (DE)**

(56) References cited:

| | |
|---|---|
| **US-A- 3 448 163** | **US-A- 3 988 384** |
| **US-A- 4 368 345** | **US-A- 4 795 734** |

- **DERWENT'S ABSTRACT, No. 93-218846/27, week 9327; & SU,A,1748854 (IND CARBON RES INST), 23 July 1992 (23.07.92), claim 1, Abstract.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 700 317 B1

## Description

The invention relates to a catalyst for the conversion, through a metathesis reaction, of a hydrocarbon mixture which contains cyclic olefins and acyclic olefins, and to the use of the catalyst for the conversion of these hydrocarbon mixtures.

In olefin chemistry, metathesis or disproportionation describes mutual exchange of carbon atoms between double-bond pairs. These reactions include olefin metathesis (OM), which can be generally depicted, for example, by the following reaction equation

$$\begin{array}{ccccccc} C & & C'' & & C & = & C'' \\ \| & + & \| & \dashrightarrow & & & \\ C' & & C'' & & C' & = & C'' \end{array}$$

In olefin metathesis, the C = C bonds thus break up and the halves recombine, thereby forming new olefins.

By olefin metathesis it is possible to prepare from acyclic olefins and cyclic or straight-chain terminal dienes hydrocarbons having the formula

$$H_2C=CH(CH_2)_mCH=CH_2 \text{ or } (CH_3)_2C=CH(CH_2)_mCH=CH_2$$

where m is greater than one, typically 2-6. Such hydrocarbons can advantageously be used as comonomers in olefin polymerization. Examples of such reactions include the conversion of 1,5-cyclo-octadiene or cyclo-octene by means of ethylene or isobutene to 1,5-hexadiene, 1,9-decadiene or 6-methyl- 1,5-heptadiene, which are usable as comonomers in special-purpose polymers. These processes are based on an olefin metathesis reaction, and the desired product is usually the reaction product lightest in weight. The crucial factor in terms of the profitability of the process is the optimization of the catalyst and the conditions so as to produce primarily the product lightest in weight.

In olefin metathesis reactions, catalyst systems are generally used which contain a compound of a transition metal, such as tungsten or rhenium, often together with a compound serving as a cocatalyst or a promotor. The reactions are in general equilibrium reactions and may with a good catalyst reach a state of equilibrium in a few seconds.

A typical metathesis catalyst is thus rhenium oxide on an alumina carrier. In the preparation of such catalysts, the active component is usually introduced onto the surface of an oxide carrier by impregnation. In impregnation, the carrier is moistened (the pores are filled) with a solution in which the active component has been dissolved. When the solvent is dried by evaporation, the active component will remain within the carrier particle. By using the solution only in the amount of the pore volume it is ensured that the compound will not crystallize in the space between the particles. However, possible crystallization inside the particles cannot thereby be prevented.

It is known that on the surface of gamma alumina in an aqueous solution there are groups $Al\text{-}OH_2^+$, Al-OH, and $Al\text{-}O^-$, the concentration of which depends, for example, on the pH of the solution. The anions attach to sites having a positive charge, for example according to Reaction 1, and cations, for example according to Reaction 2.

$$A^- + Al\text{-}OH_2^+ \rightarrow Al\text{-}O\text{-}A + H_2O \qquad (1)$$

$$K^+ + Al\text{-}O^- \rightarrow Al\text{-}O\text{-}K \qquad (2)$$

Such surface reactions may be carried out, for example, by using ion exchange techniques. Thus, for example, patent publication US 3 489 692 relates to the preparation of a catalyst on a metal carrier by ion exchange techniques. By ion exchange is generally meant the replacing of $H^+$ ions on the original carrier with metal cations in such a manner that the metal is chemically bonded to the oxide and not only mixed with the oxide. According to the patent, the surface of an oxide containing hydrogen ions is first treated with an alkaline solution to replace the hydrogen ions with alkali metal cations. These are thereafter replaced with iron, cobalt or nickel cations by treating the carrier with a solution which contains said cations. Thereafter the carrier is treated in reducing conditions to convert the exchanged metal cations to a metallic form. The obtained catalysts are used for various hydrocarbon conversion reactions, such as hydrogenation, dehydrogenation, desulfurization, hydrocracking, and steam cracking.

A similar method for introducing metal cations, such as tungsten, molybdenum and rhenium cations, onto the surface of a carrier is disclosed in GB patent 1 306 158. In this patent, the carrier used is a crystalline zeolite material,

and the ion exchange takes place from non-aqueous solutions of the said cations.

It is known that isobutene is easily dimerized by known metathesis catalysts. As a consequence, isobutene is consumed in the formation of non-desirable products, and considerable difficulties arise in the purification of the product. For example, 6-methyl-1,5-heptadiene (a metathesis product of 1,5-cyclo-octadiene and isobutene) and the dimer of isobutene are not separated in conventional distillation. Furthermore, dimerization decreases diene production by the catalysts. When the dimerization activity is high, considerable amounts of other oligomers are also produced in the reaction.

Attempts have been made to eliminate the problem, for example, by impregnating the catalyst with an alkali metal salt which neutralizes the dimerization-active bonding sites on the surface of the catalyst carrier. However, thereby the metathesis activity of the catalyst is considerably decreased. Thus, for example, according to patent publication DE2604261, a catalyst is prepared which contains 5-70 % by weight of rhenium oxide ($Re_2O_7$) on gamma alumina. The catalyst additionally contains an alkali metal compound, e.g. a potassium compound, 1-5 % by weight. The patent includes no description of the potassium compounds used or of the method by which they are added.

Patent publication GB1216587 discusses in particular the polymerization propensity due to olefins, and in particular alkaline olefins such as isobutene, and its elimination by the modification of the total acidity and the distribution of the acid concentration on the surface of alumina. According to the patent, anions usable for the modification of alumina include anions, such as vanadate, fluoride and phosphate, derived from mineral acids and their salts. According to the patent, together with the said anion it is possible to use modifiers which decrease the activity of alumina in regard to polymerization reactions. Such modifiers include alkali metal ions and earth-alkali metal ions. However, no examples of the use of such modifiers are presented in the patent.

US-A-3,448,163 discloses a process for disproportionating olefins by impregnating alumina with a solution of rhenium heptoxide in an organic solvent which may be an aliphatic alcohol. The organic solvent is inert.

US-A-4,368,345 is directed to a catalyst for the disproportionation of n-olefins. The catalyst is prepared by impregnating a silica support with an alcoholic solution of a halide of a metal selected from molybdenum and tungsten and thereafter removing the alcoholic solvent by evaporation and performing a heat treatment at an elevated temperature.

US-A-4,795,734 discloses a process for manufacturing a rhenium-containing catalyst comprising dry impregnation of an alumina-containing porous carrier and thereafter performing a first and second heat treatment step. A catalyst prepared in the above way, is used for producing n-olefins by metathesis. According to this disclosure it is desirable to remove almost the entire amount of the solvent by the second heat treatment step.

According to the present invention it has been observed that the above-mentioned known methods do not provide an optimal solution to the preventing of the dimerization propensity of isobutene in the preparation of dienes from cyclic olefins by a metathesis reaction. Thus, for example, the use of earth-alkali metal salts decreases the dimerization of isobutene, but at the same time the activity of the catalyst in terms of the desired end products is drastically decreased.

Thus, according to the invention, a catalyst is produced for the conversion of a hydrocarbon mixture which contains isobutene and cyclic olefins or straight-chain terminal dienes by means of a metathesis reaction for the preparation of diene hydrocarbons, which catalyst is prepared by adding a rhenium and/or molybdenum compound onto an inorganic oxide carrier and by calcining the catalyst thus obtained. The catalyst according to the invention is characterized in that, before the adding of the rhenium or molybdenum compound, the carrier is treated with an alcohol which serves as a modifier decreasing the polymerization activity of the catalyst.

The carrier used in a catalyst according to the invention is an aluminum oxide or silica-alumina carrier. The aluminum oxide is alumina or some other inorganic carrier which contains aluminun, such as aluminum hydrate. The carrier preferably has a narrow pore size distribution and preferably an average pore size of 3-15 nm.

The active-metal source used in a metathesis catalyst according to the invention consists of water-soluble compounds of rhenium or molybdenum, which in an aqueous solution form rhenium- or molyodenum-containing anions. In the case of rhenium, a suitable water-soluble compound is perrhenic acid. In the case of molybdenum, a suitable molybdenum compound is ammonium molybdate. The aqueous solution used for the treatment of the carrier may also contain rhenium, and molybdenum compounds at the same time for the preparation of mixed catalysts.

The concentration of the rhenium or molybdenum compound can be selected according to the desired active-metal concentration in the final catalyst and according to the number of surface bonding sites on the carrier, which can be affected by the selection of the pH of the aqueous solution.

The adding of the rhenium or molybdenum compound is preferably carried out by an ion exchange method, in which case the carrier is treated with an aqueous solution of the active metal, and after the treatment the carrier is washed with water. In this case only an active metal amount corresponding to the vacant surface bonding sites is left in the catalyst. The number of surface bonding sites can be regulated by means of the pH.

According to the invention, before the adding of the active metal the carrier is treated with a modifier which inhibits the dimerization of isobutene when the catalyst is used for a metathesis reaction. The modifiers used are certain

compounds which on the one hand increase the activity of the catalysts, i.e. the degree of conversion of the initial substances to the desired end products, and on the other hand effectively inhibit isobutene dimerization.

The modifier used in a catalyst according to the invention consists of alcohols. Modifiers suitable for use in the preparation of catalysts according to the invention include straight-chain and branched-chain alcohols such as methanol, ethanol, propanol, isopropanol, butanol and glycerol. The alcohol can be added to the carrier as such or out of an aqueous solution. The amount or concentration of the alcohol is not of importance as such, as long as it is present in ample excess in relation to the surface bonding sites reacting with it. After the treatment the excess of the alcohol is removed, preferably by washing with water.

The precise mechanism of the action of the alcohol is not known, but it evidently reacts selectively with the carrier and is effective in spite of that the carrier is washed with water after the alcohol treatment. It is possible that, after the breaking off of the water, the hydrocarbon radical of the alcohol attaches permanently to the alumina surface, thereby eliminating the acid site corresponding to the oligomerization of i-butene.

After the adding of the modifier the carrier may be dried before the adding of the active metal, or the active metal may be added directly without a preceding intermediate drying.

After the adding of the active metal, the catalyst is calcined by heating it in an inert atmosphere or in air. A suitable calcination temperature is usually within the range 400-900 °C, and a suitable time within the range 0.5-6 hours.

The metathesis reaction can be performed by using a catalyst according to the invention either as a batch reaction or as a continuous reaction by contacting the feed materials in a fluidized-bed or fixed-bed reactor. The recommendable reaction conditions, such as temperature, pressure and flow rates, may vary depending on the feed materials used and the desired end products, but in general the reaction is carried out at a temperature of 20-150 °C at a pressure of 0-2000 bar.

When so desired, is possible to use as diluents in the reaction paraffinic or cycloparaffinic hydrocarbons, such as propane, cyclohexane, methylcyclohexane, pentane, hexane, heptane, iso-octane and dodecane, provided that they will not have a detrimental effect on the metathesis reaction. Aromatic hydrocarbons such as toluene may also be used.

In a continuous process the suitable reaction time depends mainly on the activity of the catalyst used. When a catalyst having a higher activity is used, the reaction time is usually shorter than with a catalyst having a lower activity. It is also be to be noted that longer contact times may lead to undesirable secondary reactions and to the formation of non-desirable components, and therefore shorter reaction times are recommended.

A catalyst according to the invention can be used for the conversion of hydrocarbons containing cyclic olefins or diolefins and acyclic olefins to diene hydrocarbons. Cyclic olefins or diolefins are hydrocarbons in which the number of carbon atoms is up to 14. These olefins or diolefins may be nonsubstituted or substituted. Examples of suitable cycle olefins and diolefins include cyclobutene, cyclopentene, cyclohexene and cyclo-octene, as well as cyclo-octadiene. Suitable acyclic olefins include ethylene, propylene, butenes, pentenes, and hexenes. The aim is to maintain the conversion of acyclic olefin as high as possible in order for the selectivity of the reaction to remain as high as possible. Hydrocarbon mixtures suitable for being converted by means of the catalyst according to the invention include hydrocarbon mixtures which contain ethylene or butene and cyclo-octadiene or cyclo-octenes. The catalyst according to the invention is especially suitable for the conversion of 1,5-cyclo-octadiene, cyclo-octene or cyclopentene with the help of ethylene or isobutene to hexadiene, decadiene or methylheptadiene. These can advantageously be used as monomers in the preparation of special-purpose polymers.

The invention is described below with reference to the examples presented. The catalysts were prepared on an alumina carrier (Akzo 000-1.5E, 190 $m^2/g$, particle size 0.25-0.5 mm) by using impregnation or adsorption by the ion exchange technique.

In impregnation, the alumina was impregnated with an aqueous solution of the impregnation substance, roughly in an amount corresponding to the pore volume of the alumina.

In ion exchange, an excess of an aqueous solution of the ion-exchange alcohol, or alternatively pure alcohol, was poured over the alumina.

In each case, the sample was allowed to stand at room temperature in a closed vessel for a number of hours, but fewer than 24 hours. In the ion exchange method the sample was thereafter washed with water, unless otherwise mentioned. The sample was dried at 120 °C and was calcined with air at 600 °C for 3 hours and with nitrogen for 0.5 hours, and was cooled in nitrogen. The storing and the charging into the reactor took place in nitrogen gas.

All the element determinations were made on a completed catalyst.

The testing of the catalysts in the metathesis reaction of 1,5-cyclo-octadiene (COD) and isobutene (IB) was conducted, unless otherwise mentioned, in a dodecane solution at room temperature at an initial pressure of approx. 10 bar. At the beginning of the test the reaction vessel contained isobutene in such an amount that the ratio IB (mol) / COD (mol) was approx. 11 and 1,5-cyclo-octadiene in such an amount that the ratio COD (g) / catalyst (g) was 2.2. The amount of catalyst in the metathesis reaction was 4.0 g. The dodecane contained heptane 12 % as an internal standard. The composition of the reaction solution was analyzed by gas chromatography.

The testing of the catalysts in the metathesis reaction of cyclopentene (CP) and isobutene (IB) was conducted, unless otherwise mentioned, in a dodecane solution at room temperature at an initial pressure of approx. 10 bar. At the beginning of the test the reaction vessel contained isobutene in such an amount that the ratio IB (mol) / CP (mol) was 15, and cyclopentene in such an amount that the ratio CP (g) / catalyst (g) was approx. 1.6. Catalyst was present in an amount of 3.4-4 g. The dodecane contained hexadecane 4 % as an internal standard. The composition of the reaction solution was analyzed by gas chromatography.

Example 1 (comparison)

In this example, the inhibition of isobutene dimerization was studied in the metathesis reaction of 1,5-cyclooctadiene and isobutene.

Catalyst 1 was prepared by the adsorption of $HReO_4$ by the ion exchange method out of an 0.2 M aqueous solution. Catalyst 2 was prepared by impregnating alumina first with a 3.7 % aqueous solution of $K_2CO_3$ and then with an 0.85 M aqueous solution of $HReO_4$.

A metathesis reaction was carried out in a toluene solution at 70 °C by using the ratio IB (mol) / COD (mol) = 4.4 and COD (g) / catalyst (g) = 2.0. The initial pressure of isobutene was 12 bar. The catalyst was used in an amount of 4.0 g. The results are shown in accompanying Table 1.

Table 1

| Catalyst | K (%) | Re (%) | Methylheptadiene 2 h (g) | Dimer 2 h (g) |
|---|---|---|---|---|
| 1 (comparison) | - | 5.8 | 1.2 | 2.5 |
| 2 (comparison) | 1.0 | 6.9 | 0.6 | 0.08 |
| 2 h (g) = the amount of formed methylheptadiene and the amount of the dimer (dimer of methylheptadiene or isobutene dimer) at 2 hours from the starting of the reaction | | | | |

When the catalyst was prepared in accordance with DE2604261, dimerization decreased but at the same tine production dropped significantly (Catalyst 2).

Example 2

In this example, the inhibition of isobutene dimerization was studied in the metathesis reaction of 1,5-cyclooctadiene and isobutene by using catalysts treated with various alcohols.

Catalyst 3 (comparison catalyst) was prepared by adsorption onto alumina out of an 0.1 M aqueous solution of $HReO_4$ by the ion exchange method. Catalyst 4 (comparison catalyst) was prepared by adsorption of HCl onto alumina out of an 0.1 M aqueous solution by the ion exchange method, by washing off the excess with water, and then by adsorption of $HReO_4$ out of an 0.1 M aqueous solution by ion exchange. The completed catalyst contained 0.57 % Cl.

The catalysts 5-12 according to the invention were prepared by treating the alumina first with alcohol, by washing with water, and then by attaching $HReO_4$ by the ion exchange method (Al (g) / Re (g) = 7-11). The results are shown in accompanying Table 2.

Table 2

| Catalyst | Treatment | Re (%) | Conversion 2 h (%) | Dimer 22 h (g) |
|---|---|---|---|---|
| 3 (comp.) | - | 5.4 | 59 | 2.2 |
| 4 (comp.) | - [1)] | 3.8 | 88 | 5.5 |
| 5 | ethanol [2)] | 2.7 | 87 | 0.07 |
| 6 | methanol | 3.1 | 87 | 0.05 |
| 7 | 1-propanol | 2.9 | 89 | 0.04 |
| 8 | 2-propanol | 2.8 | 87 | 0.05 |
| 9 | 1-butanol | 3.1 | 88 | 0.04 |
| 10 | ethylene glycol | 2.5 | 89 | 0.05 |
| 11 | glycerol | 2.4 | 84 | 0.04 |
| 12 | 2-methyl-1-propanol | 2.9 | 87 | 0.04 |

1) Cl concentration 0.57 %
2) absolute ethanol, no washing

When alcohols are adsorbed onto alumina by the ion exchange method, the catalyst activity rises to a high level and the dimerization propensity almost disappears. With the comparison catalysts, the dimerization propensity was considerably high.

Example 3

In this example, the inhibition of isobutene dimerization was studied in the metathesis reaction of cyclopentene and isobutene by using a catalyst treated with ethanol.

The catalysts were prepared by treating alumina with ethanol, by washing with water, and then by attaching $HReO_4$ by the ion exchange method (Al (g) / Re (g) = 7-11). The results are shown in accompanying Table 3.

Table 3

| Catalyst | Treatment | Re (%) | Conversion 2 h (%) | Dimer 22 h (g) |
|---|---|---|---|---|
| 13 | EtOH [1)] | 2.9 | 69 | 0.03 |
| 14 | EtOH [2)] | 2.7 | 61 | 0.02 |

1) absolute ethanol, no washing
2) 22 h; 0.19 g

Example 4

In this example, the inhibition of cyclopentene polymerization was studied in the metathesis reaction of cyclopentene and ethylene by using a catalyst treated with ethanol.

The catalyst 15 (comparison) was prepared according to the known technique by impregnating onto alumina 0.27 M $K_2CO_3$ solution, by drying at 120 °C and by impregnating with 0.85 M $HReO_4$ solution.

The catalyst 16 was prepared by treating alumina with absolute ethanol, by decanting the excess of ethanol off and by attaching then $HReO_4$ by the ion exchange method (Al (g) / Re(g) = 11).

Cyclopentene (CP) was allowed to react in the presence of the catylysts at room temperature in a pressure of about 45 bar.

The results are shown in accompanying Table 4.

Table 4

| Catalyst | Treatment | Re (%) | K (%) | CP (g/g cat) | Conversion 2 h (%) | Heptadiene 20 h (g/g cat) |
|---|---|---|---|---|---|---|
| 15(comp.) | $K_2CO_3$ | 8.2 | 0.92 | 48[1] | 32 | 0.0 |
| 16 | EtOH | 3.2 | - | 1.3[2] | 98 | 1.1 |

1) 20 % cyclopentene in the starting solution, solvent toluene
2) 14 % cyclopentene in the starting solution, solvent dodecane

A small amount of comparison catalyst was able to polymerize a considerable amount of cyclopentene and the desired metathesis product 1,6-heptadiene was not observed. The catalyst treated with ethanol produced 1,6-heptadiene with good yield.

**Claims**

1. A catalyst for the conversion of a hydrocarbon mixture which contains acyclic olefins and cyclic olefins or straight-chain terminal dienes by a metathesis reaction to prepare diene hydrocarbons, which catalyst is prepared by adding rhenium and/or molybdenum compound onto an inorganic oxide carrier and by calcining the catalyst thus obtained, characterized in that before the adding of the rhenium or molybdenum compound the carrier is treated with an alcohol which serves as a modifier decreasing the polymerization activity of the catalyst.

2. A catalyst according to claim 1, characterized in that the said modifier is added onto the carrier out of an aqueous solution or without a solvent and that the excess of the modifier is removed before the adding of the rhenium or molybdenum compound.

3. A catalyst according to Claim 1, **characterized in that** the said modifier is a mono or multivalent aliphatic alcohol containing 1-4 carbon atoms.

4. A catalyst according to Claim 3, **characterized in that** the alcohol is methanol, ethanol, propanol, isopropanol, butanol, glycerol, ethylene glycol, isobutyl alcohol, or mixtures thereof.

5. A catalyst according to any of the above claims, **characterized in that** the said rhenium or molybdenum compound is added onto the carrier out of an aqueous solution of perrhenic acid and/or of ammonium molybdate.

6. The use of a catalyst according to any of the above claims for the conversion by a metathesis reaction of hydrocarbons which contain cyclic olefins or diolefins and acyclic olefins to prepare diene hydrocarbons.

7. The use according to Claim 6, **characterized in that** the cyclic olefin or diolefin is selected from the group cyclobutene, cyclopentene, cyclohexene, cyclo-octene, and cyclo-octadiene.

8. The use according to Claim 6 or 7, **characterized in that** the acyclic olefin is selected from the group ethylene, propylene, butenes, pentenes, and hexenes.

**Patentansprüche**

1. Katalysator für die Umsetzung einer Kohlenwasserstoffmischung, die acyclische Olefine und cyclische Olefine oder geradkettige, terminale Diene enthält, durch eine Metathesereaktion zur Herstellung von Dienkohlenwasserstoffen, wobei der Katalysator durch Zugabe einer Rhenium- und/oder Molybdänverbindung auf einen anorganischen Träger und durch Calcinierung des so erhaltenen Katalysators hergestellt ist, dadurch gekennzeichnet, daß der Träger vor der Zugabe der Rhenium- oder Molybdänverbindung mit einem Alkohol behandelt ist, der als ein die Polymerisationsaktivität des Katalysators senkendes Modifiziermittel dient.

2. Katalysator nach Anspruch 1, dadurch gekennzeichnet, daß das Modifiziermittel auf den Träger aus einer wäßrigen Lösung oder ohne Lösungsmittel zugegeben ist, und daß der Überschuß des Modifiziermittels vor der Zugabe der Rhenium- oder Molybdänverbindung entfernt ist.

3. Katalysator nach Anspruch 1, dadurch gekennzeichnet, daß das Modifiziermittel ein ein- oder mehrwertiger, 1 bis 4 Kohlenstoffatome enthaltender, aliphatischer Alkohol ist.

4. Katalysator nach Anspruch 3, dadurch gekennzeichnet, daß der Alkohol Methanol, Ethanol, Propanol, Isopropanol, Butanol, Glycerin, Ethylenglykol, Isobutylalkohol oder Mischungen davon ist.

5. Katalysator nach einem der vorstehenden Ansprüche, dadurch gekennzeichnet, daß die Rhenium- oder Molybdänverbindung auf den Träger aus einer wäßrigen Lösung von Perrheniumsäure und/oder Ammoniummolybdat zugegeben ist.

6. Verwendung eines Katalysators nach einem der vorstehenden Ansprüche für die Umsetzung durch eine Metathesereaktion von Kohlenwasserstoffen, die cyclische Olefine oder Diolefine enthalten, und acyclischen Olefinen zur Herstellung von Dienkohlenwasserstoffen.

7. Verwendung nach Anspruch 6, dadurch gekennzeichnet, daß das cyclische Olefin oder Diolefin aus der Gruppe Cyclobuten, Cyclopenten, Cyclohexen, Cycloocten und Cyclooctadien ausgewählt wird.

8. Verwendung nach Anspruch 6 oder 7, dadurch gekennzeichnet, daß das acyclische Olefin aus der Gruppe Ethylen, Propylen, Butenen, Pentenen und Hexenen ausgewählt wird.

**Revendications**

1. Catalyseur destiné à la conversion d'un mélange d'hydrocarbures qui contient des oléfines acycliques et des oléfines cycliques ou des diènes terminaux à chaîne linéaire par une réaction de métathèse pour préparer des hydrocarbures diéniques, lequel catalyseur est préparé par addition d'un composé rhénium et / ou molybdène sur un support d'oxyde minéral et en calcinant le catalyseur ainsi obtenu, caractérisé en ce qu'avant d'ajouter le composé rhénium ou molybdène, le support est traité avec un alcool qui sert d'agent modificateur réduisant l'activité de polymérisation du catalyseur.

2. Catalyseur selon la revendication 1, caractérisé en ce que ledit agent modificateur est ajouté sur le support à partir d'une solution aqueuse ou sans solvant et en ce que l'excédent de l'agent modificateur est enlevé avant l'addition du composé rhénium ou molybdène.

3. Catalyseur selon la revendication 1, caractérisé en ce que l'agent modificateur est un alcool aliphatique mono ou multivalent contenant 1 - 4 atomes de carbone.

4. Catalyseur selon la revendication 3, caractérisé en ce que l'alcool est le méthanol, l'éthanol, le propanol, l'isopropanol, le butanol, le glycérol, l'éthylène - glycol, l'alcool d'isobutyle ou leurs mélanges.

5. Catalyseur selon les revendications ci-dessus, caractérisé en ce que ledit composé rhénium ou molybdène est ajouté sur le support à partir d'une solution aqueuse d'acide perrhénique et / ou de molybdate d'ammonium.

6. Utilisation d'un catalyseur selon l'une quelconque des revendications ci-dessus pour la conversion par une réaction de métathèse des hydrocarbures qui contiennent des oléfines cycliques ou des dioléfines et des oléfines acycliques pour préparer des hydrocarbures diéniques.

7. Utilisation selon la revendication 6, caractérisée en ce que l'oléfine cyclique ou la dioléfine sont choisies dans le groupe cyclobutène, cyclopentène, cyclohexène, cyclo - octène et cyclo - octadiène.

8. Utilisation selon les revendications 6 ou 7, caractérisée en ce que l'oléfine acyclique est choisie dans le groupe éthylène, propylène, butènes, pentènes et hexènes.